# EUROPÄISCHE PATENTSCHRIFT

(11) **EP 2 307 430 B1**
(45) Veröffentlichungstag und Bekanntmachung des Hinweises auf die Patenterteilung: **27.08.2014**
(21) Anmeldenummer: 09780655.8
(22) Anmeldetag: 15.07.2009
(51) Int. Cl.: C07F 15/00, H01L 51/00, H05B 33/00

(54) **PHOSPHORESZENTE METALLKOMPLEXVERBINDUNG, VERFAHREN ZUR HERSTELLUNG DAZU UND STRAHLUNGSEMITTIERENDES BAUELEMENT**
PHOSPHORESCENT METAL COMPLEX COMPOUND, METHOD FOR THE PREPARATION THEREOF AND RADIATING COMPONENT
COMPOSÉ COMPLEXE MÉTALLIQUE PHOSPHORESCENT, PROCÉDÉ DE FABRICATION ASSOCIÉ ET COMPOSANT ÉMETTEUR DE RAYONNEMENT

(30) Priorität: 18.07.2008 DE 102008033929
(43) Veröffentlichungstag der Anmeldung: 13.04.2011
(73) Patentinhaber: OSRAM GmbH, 80807 München (DE)
(72) Erfinder: DE COLA, Luisa, 48147 Münster (DE); HARTMANN, David, 91056 Erlangen (DE); SARFERT, Wiebke, 91074 Herzogenaurach (DE); SCHMID, Günter, 91334 Hemhofen (DE)
(74) Vertreter: Epping - Hermann - Fischer
(86) Internationale Anmeldenummer: PCT/EP2009/059092
(87) Internationale Veröffentlichungsnummer: WO 2010/007107

(56) Entgegenhaltungen:
- WO-A-03/018653
- WO-A-2006/135076
- US-A1- 2007 001 166
- CHEN ET AL: "Blue Phosphorescent Heteroleptic Triscyclometallated Ir(III) Organometallic Complexes" 22TH. INTERNATIONAL CONFERENCE ON ORGANOMETALLIC CHEMISTRY ICOMC 2006), BOOK OF ABSTRACTS, POSTER PRESENTATIONS, ZARAGOZA, JULY 23-28, 2006,, Bd. 2, 23. Juli 2006 (2006-07-23), Seite P662, XP002510842
- LAMANSKY S ET AL: "Synthesis and Characterization of Phosphorescent Cyclometalated Iridium Complexes" INORGANIC CHEMISTRY, AMERICAN CHEMICAL SOCIETY, EASTON, US, Bd. 40, Nr. 7, 1. Januar 2001 (2001-01-01), Seiten 1704-1711, XP002196399 ISSN: 0020-1669

## Beschreibung

Die Erfindung betrifft eine phosphoreszente Metallkomplexverbindung, Verfahren zur Herstellung dazu und ein strahlungsemittierendes Bauelement, insbesondere eine lichtemittierende organische elektrochemische Zelle (organic light emitting electrochemical cell OLEEC).

Die Druckschrift Chen et al. (22th. International Conference On Organometallic Chemistry ICOMC 2006, Book of Abstracts, Poster, Presentations, Bd. 2, 23. Juli 2006) betrifft blau phosphorezierende heteroleptische Triscyclometallasisierte Ir(III) Organometallkomplexe.

Die Druckschrift US 2007/001166 betrifft phosphorezente Osmium (II) Komplexe.

Die Druckschrift Lamanskys et al. (Inorganic Chemistry, American Chemical Society, Easton, Bd. 40, Nr. 7, 1. Januar 2001) betrifft phosphorezente cyclometallisierte IridiumKomplexe.

Die Druckschrift WO 03/018653 betrifft neutrale phosphorezente Komplexe.

Die Druckschrift WO 2006/135076 betrifft lichtemittierende Polymermaterialien.

Ganz generell haben organische elektrolumineszente Elemente zumindest eine organische Schicht, die sich zwischen zwei Elektroden befindet. Sobald Spannung an die Elektroden angelegt wird, werden Elektronen von der Kathode in die untersten unbesetzten Molekülorbitale der organischen Licht emittierenden Schicht injiziert und wandern auf die Anode zu. Korrespondierend dazu werden Löcher von der Anode in die obersten besetzten Molekülorbitale der organischen Schicht injiziert und wandern entsprechend zur Kathode. In den Fällen, wo sich wanderndes Loch und wanderndes Elektron innerhalb der organischen Licht emittierenden Schicht auf einem lichtemitterenden Stoff treffen, entsteht ein Exciton, das unter Lichtemission zerfällt. Damit das Licht überhaupt aus dem elektrolumineszierenden Element austreten kann, muss zumindest eine Elektrode transparent sein, in den meisten Fällen ist das eine Elektrode aus Indium-Zinn-Oxid, die als Anode eingesetzt wird. Die ITO-Schicht wird normalerweise auf einem Glasträger abgeschieden.

In den organischen Licht emittierenden Dioden (OLEDs) wird, insbesondere bei den mit so genannten small molecules aufgebauten OLEDs, ein so genannter Multilayer-Aufbau realisiert, weil zusätzlich zu der Licht emittierenden Schicht auch noch effizienzerhöhende Schichten wie Loch- und/oder Elektroneninjektionsschichten zwischen den Elektroden zum besseren Übergang der Ladungsträger angeordnet werden. Oftmals werden dabei hochreaktive Materialien eingesetzt, so dass für die Lebensdauer des licht emittierenden Elements unter anderem die Verkapselung eine entscheidende Rolle spielt, da sie die Hilfsschichten vor Zersetzung bewahrt.

Alternativ dazu gibt es die so genannten organischen Licht emittierenden elektrochemischen Zellen (OLEECs) die einfacher als die OLEDs aufgebaut sind und die in den meisten Fällen durch ein einfaches Einbringen einer organischen Schicht zwischen zwei Elektroden und nachfolgendes Verkapseln realisierbar ist. Die aktive Schicht einer OLEEC ist in der Regel aus einem Material, das eine Mischung aus einem Ionenleiter/Elektrolyten oder auch einer völlig inerten Matrix (Isolator) mit einer emittierenden Spezies ist. Dafür geeignet sind ionische Übergangsmetallkomplexe (ionisized transistion metal complexes, kurz: iTMC), wie beispielsweise Ruthenium-tris-bipyridin-hexafluorophosphate in polymeren Matrizen. Es gibt jedoch noch keine ausreichende Auswahl an geeigneten Materialien, insbesondere mangelt es an Materialien, die blau emittieren.

Aufgabe der vorliegenden Erfindung ist es daher, eine Materialklasse zu schaffen, die für den Einsatz in OLEEC-Zellen geeignet ist, sowie eine Synthese dazu anzugeben, des weiteren ist es Aufgabe der Erfindung eine OLEEC-Zelle anzugeben, die unter Verwendung der Materialklasse aufgebaut ist sowie die Verwendung der Materialklasse in OLEEC-Zellen.

Der Gegenstand der Erfindung und die Lösung der Aufgabe werden durch die Ansprüche, die Beschreibung, und die Figuren offenbart.

Entsprechend ist Gegenstand der Erfindung eine lichtemittierende organische elektrochemische Zelle umfassend
- ein Substrat,
- eine erste Elektrodenschicht auf dem Substrat,
- zumindest eine organische emittierende Schicht auf der ersten Elektrodenschicht und
eine zweite Elektrodenschicht auf der organischen emittierenden Schicht, wobei die organische emittierende Schicht eine phosphoreszente Metallkomplexverbindung umfasst, die die Strukturformel 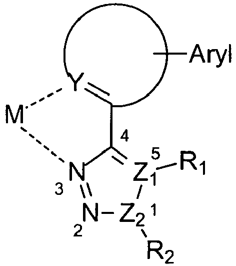 aufweist, wobei gilt:
M=Ir, Re, Os, Pt, Au, Hg, Ru, Rh, Pd, Ag, Cu
Y, Z₁, Z₂ = N oder C
R₁, R₂ = unabhängig voneinander - H, verzweigte Alkylreste, unverzweigte Alkylreste, kondensierte Alkylreste, ringförmige Alkylreste, vollständig oder teilweise substituierte unverzweigte Alkylreste, vollständig oder teilweise substituierte verzweigte Alkylreste, vollständig oder teilweise substituierte kondensierte Alkylreste, vollständig oder teilweise substituierte ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene, wobei R₁ und/oder R₂ geladen sind, und damit die phosphoreszente Metallkomplexverbindung geladen ist,
   und
Aryl = ein beliebiger, teilweise oder vollständig substituierter aromatischer oder heteroaromatischer Rest ist, der auch kondensiert sein kann, eine Brücke zu einer weiteren Verbindung knüpfen kann, und/oder kondensiert oder anneliert mit weiteren Aromaten oder Heteroaromaten, sowie verbunden mit weiteren cyclischen Verbindungen, vorliegen kann.

Schließlich ist Gegenstand der Erfindung ein Verfahren zur Herstellung einer lichtemittierenden organischen elektrochemischen Zelle beinhaltend die folgenden Verfahrensschritte zur Herstellung einer phosphoreszenten Metallkomplexverbindung
A) Bereitstellen einer Zentralatomverbindung eines metallischen Zentralatoms, aufweisend an das Zentralatom koordinierte Austauschliganden,
B) Mischen der Zentralatomverbindung und eines in einem ersten Lösungsmittel gelösten Liganden zur Bildung der Metallkomplexverbindung, wobei der Austauschligand durch den Liganden, der zweizähnig am Zentralatom koordiniert und eine Triazoleinheit umfasst, ersetzt wird.

Insbesondere handelt es sich um eine Materialklasse eines Metallkomplexes der folgenden allgemeinen Struktur I:

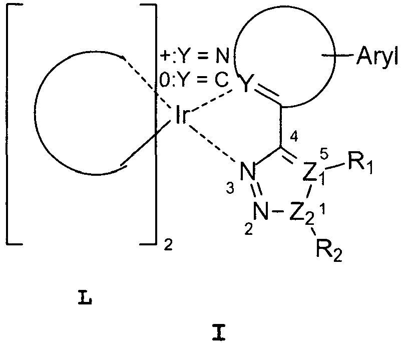

Dabei hat der Komplex zwei bekannte Liganden L(links dargestellt), die unabhängig voneinander ausgewählt werden können und gleich oder ungleich sein können und vorzugsweise zweizähnig komplexieren, insbesondere über einen Kohlenstoff und ein Stickstoffatom, wobei diese bekannten Liganden L beispielsweise nach einer Ausführungsform der Erfindung die klassischen und auch handelsüblichen Emitter mit Phenylpyridinliganden, zum Blaushift beispielsweise mit Fluor substituiert, sind. Bekannte Komplexe mit Iridium als Zentralatom sind das 2,4-difluorophenyl-2-pyridyl-Iridium (III) -picolinate (FIrPic) oder FIr₆.

Nach einer weiteren Ausführungsform der Materialklasse sind die beiden links vom Metallatom gezeigten und bereits Literatur bekannten Liganden L vorzugsweise ausgewählt aus den folgenden Dokumenten: WO 2005/097942 A1, WO 2006/013738 A1, WO 2006/098120A1, WO 2006/008976 A1, WO 2005/097943 A1, WO 2006/008976 A1 (Konica Minolta) oder US 6,902,830, US 7,001,536, US 6,830,828, WO 2007/095118 A2, US 2007 0 190 359 A1 (UDC), EP 1 486 552 B1, beispielhaft genannt seien dabei das 2-Phenyl-pyridin oder das 2-Phenylimidazol sowie verwandte und ähnliche Strukturen, wie beispielsweise das Phenanthridin.

Nach weiteren vorteilhaften Ausführungsformen können die beiden bekannten Liganden L beispielsweise über eine Carbenfunktionalität verfügen, die als Quelle tiefer blauer Emission dient. Beispiele für diese Liganden L sind in den Veröffentlichungen WO 2005/19373 or EP 1 692 244 B1 zu finden.

Weitere Beispiele möglicher Liganden L sind aus den Veröffentlichungen EP 1 904 508 A2, WO 2007/004113 A2, WO 2007/004113 R4A3 bekannt, wobei diese Liganden L auch im Rahmen von geladenen Metallkomplexen, die zumindest einen Phenylpyridin Ligand mit entsprechenden Donorgruppen wie Dimethylamino haben, gezeigt werden. Diese Verbindungen zeigen ein erhöhtes LUMO Niveau des Komplexes, wobei Akzeptorgruppen wie beispielsweise 2,4 Difluoro, in den Phenylring eingeführt werden, um das Niveau des HOMO-Orbitals zu erniedrigen. Es wird gezeigt, dass man durch die Variation der Liganden und deren Substituenten die Emissionsfarbe durch das ganze sichtbare Spektrum hindurch variieren kann.

Zusätzlich zu den Liganden L hat der Metallkomplex gemäß der Strukturformel I zumindest einen Triazol-Liganden, entweder ein 1,2,3- oder ein 1,2,4-Triazol. Die Triazol-Einheit hat in Ortho-Position zu den zwei sich benachbarten Stickstoffen des Triazol-Ringes einen heteroaromatischen oder einen aromatischen Substituenten. Somit entsteht eine Struktur der allgemeinen Formel I.

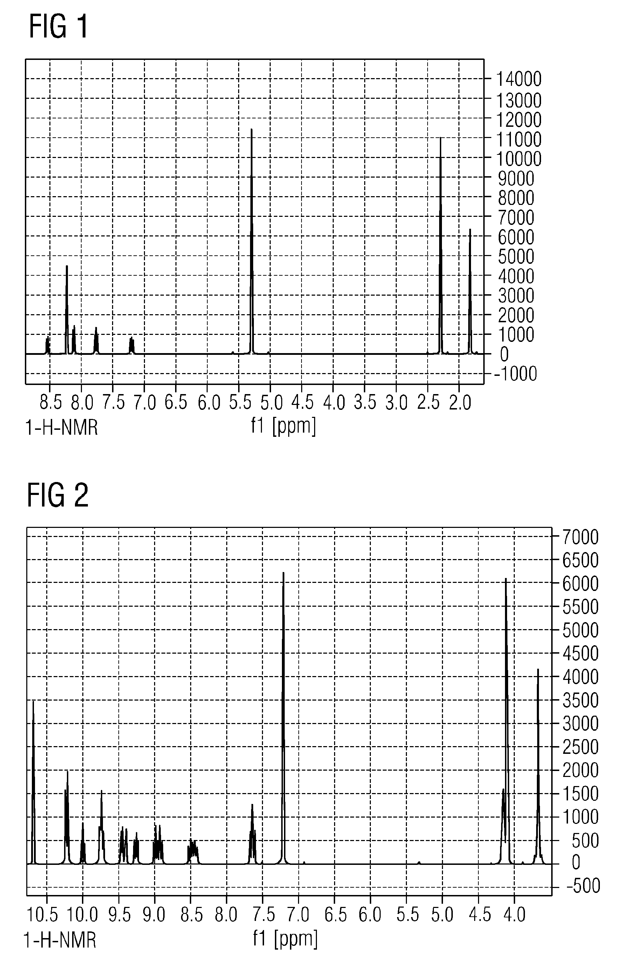

Die 1,2,3-Triazol-Verbindungen werden erhalten mit Z₂= N und Z₁= C, wie in Figur 2a gezeigt, wohingegen die 1,2,4-Triazol-Verbindungen bei Z₂= C und Z₁= N entstehen. Das Ring-Nummerierungssystem wurde anhand der 1,2,3-Triazole entwickelt und wird im Sinne der vorliegenden Beschreibung wie gezeigt verwendet. Dabei werden offensichtlich die 1,2,4 Triazole aus den 1,2,3-Triazolen durch Austausch der C und N Substituenten Z erhalten. In beiden Fällen wird das Kohlenstoffatom, das den Substituenten, der die Zweizähigkeit des gesamten Liganden bewirkt und der bevorzugt ein Arylsubstituent ist, mit 4 nummeriert.

Bevorzugt ist M = Iridium. Möglich sind aber auch Metalle, wie Re, Ru, Rh, Os, Pd, Pt, Au, Hg und Cu. Die Stöchiometrie der entsprechenden Komplexe wird dann je nach Koordinationssphäre des jeweiligen Zentralatoms variieren, insbesondere deshalb, weil nicht alle Metalle oktaedrische Komplexe wie das Iridium bilden.

Bevorzugt ist Y gleich Stickstoff. Damit sind die heterotriazol-Liganden neutral im Hinblick auf die innere Koordinationssphäre. Geladene Substituenten oder Substituenten, die Ladung stabilisieren können, also "chargeable" sind, können in den äußeren Positionen angebracht werden. Der heteroaromatische Ring enthält in ortho-Position zu dem Brücken-Kohlenstoffatom ein Stickstoffatom, das neben dem Stickstoffatom 2 in der Triazoleinheit, das zweite chelatisierende Atom des Liganden ist. Für den Fall dass Y = C ist, entsteht die klassische cyclometallierte Verbindung, wobei der Triazol-Ligand formal negativ geladen ist.

Damit werden für den Fall M = Ir neutrale Spezien erhalten. Optional können beide aromatischen Einheiten noch über eine zweite Brücke verbunden sein.

Nach einer anderen Ausführungsform der Materialklasse sind R₁ und/oder R₂ mit anderen Resten R₁' und/oder R₂' eines weiteren Metallkomplexes verbunden. Die verbindende Gruppe kann dabei aus den unten genannten Beispielen entnommen werden. Falls höher funktionale Verbindungsglieder gewählt werden, hat man Zugang zu höher vernetzten Komplexen bis hin zu Polymeren Komplexen. Auf der anderen Seite kann eine Brücke auch über einen der bekannten Liganden L zu einem oder mehreren weiteren Komplexen mit Liganden und Zentralatomen gebildet werden. Auch über diese Seite ist also ein Zugang zu oligomeren und polymeren Verbindungen möglich.

M kann auch Re, Os, Pt, Au, Hg sowie Ru, Rh, Pd und Ag, Cu sein.

Für den Fall, dass Y und Z₁ beide gleich N sind, entsteht die folgende Struktur Ia

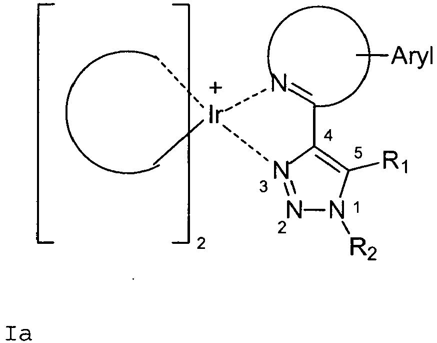

Bevorzugt umfasst die Metallkomplexverbindung nach der Erfindung eine Gruppe der Strukturformel II 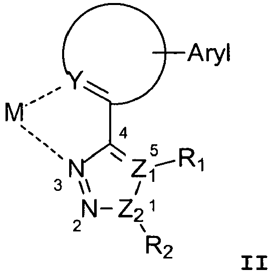 wobei
M= Ir, Re, Os, Pt, Au, Hg, Ru, Rh, Pd, Ag, Cu
Y, Z = N oder C
R = unabhängig voneinander- H, verzweigte Alkylreste, unverzweigte Alkylreste, kondensierte Alkylreste, ringförmige Alkylreste, vollständig oder teilweise substituierte unverzweigte Alkylreste, vollständig oder teilweise substituierte verzweigte Alkylreste, vollständig oder teilweise substituierte kondensierte Alkylreste, vollständig oder teilweise substituierte ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene
   und
Aryl = ein beliebiger, teilweise oder vollständig substituierter aromatischer oder heteroaromatischer Rest ist, der auch kondensiert sein kann, eine Brücke zu einer weiteren Verbindung knüpfen kann, und/oder kondensiert oder anneliert mit weiteren Aromaten oder Heteroaromaten, sowie verbunden mit weiteren cyclischen Verbindungen, vorliegen kann.

Im Folgenden werden einige Beispiele für die Ringstruktur des in ortho-Position zu den zwei benachbarten Stickstoffen des Triazolrings stehenden Heteroaromaten, beispielsweise einem 6-gliedrigen Ring, gegeben. Im einfachsten Fall handelt es sich um einen Pyridinring oder ein Derivat davon:

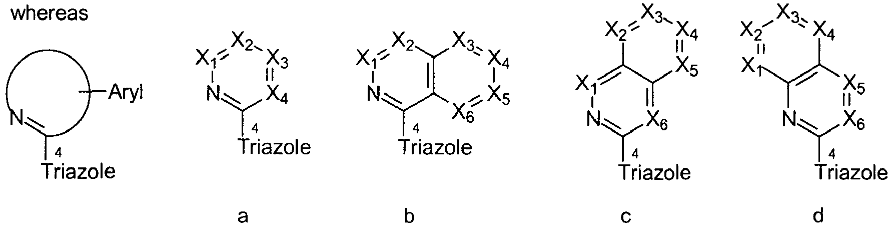

X bedeutet entweder den Rest -C-R, wobei R einer der untenstehenden Substituenten oder ein Stickstoffatom mit einem freien Elektronenpaar ist.

Beispiele für den Substituenten "a" am Triazol sind: Pyridin Derivate, wobei X₁, X₂, X₃, X₄ allesamt Reste -C - R sind, wobei alle R unabhängig voneinander und einer der untenstehenden Substituenten sind.
Pyrimidin Derivate, wobei X₂ = N oder X₄ = N, alle anderen Reste -C-R sind.
Pyrazin Derivate, wobei X₃ = N, alle anderen -C-R sind. Pyridazin Derivate, wobei X₁ = N, alle anderen -C-R sind. 1,3,5-Triazine Derivate, wobei X₂ = N and X₄ = N, alle anderen -C-R sind.

Beispiele für den Substituenten "b" am Triazol sind: Iso-chinolin Derivate, wobei alle X die Reste -C-R mit einer Verbindung zum Triazol Liganden in Position 1 sind Chinazolin Derivate, wobei X₂ = N, und alle anderen Reste vom Typ -C-R sind.
Phthalazin Derivate, wobei X₁ = N, und alle anderen Reste vom Typ -C-R sind.

Beispiele für den Substituenten "c" am Triazol sind: Isochinolin-Derivate, die strukturelle Isomere zu den Isochinolin-Derivaten der oben zu den Substituenten "b" am Triazol genannten Derivaten sind.

Beispiele für den Substituenten "d" am Triazol sind: Chinolin Derivate, wobei alle X Reste vom Typ -C-R sind. Chinoxaline Derivate, wobei X₅ = N ist und alle anderen des Typs -C-R sind,
Chinazoline Derivate mit X₆ = N ist und alle anderen Reste vom Typ -C-R sind.

Höher kondensierte Systeme können analog hergestellt werden, beispielsweise Pteridin, Acridin, Phenazin, Phenanthridin und/oder Purin und Derivate davon sowie Verbindungen mit zusätzlichen Heteroatomen wie Sauerstoff oder Schwefel in dem kondensierten Ring, der das koordinierende Stickstoffatom trägt.

Im Folgenden werden einige Beispiele für die Ringstruktur des in ortho-Position zu den zwei benachbarten Stickstoffen des Triazolrings stehenden Heteroaromaten, beispielsweise einem 5-gliedrigen Ring, gegeben:

Im einfachsten Fall ist wieder der 6-gliedrige Ring ein Pyridinring. Hier werden Beispiele für Hetero-Fünfring substituierte Triazole gegeben:

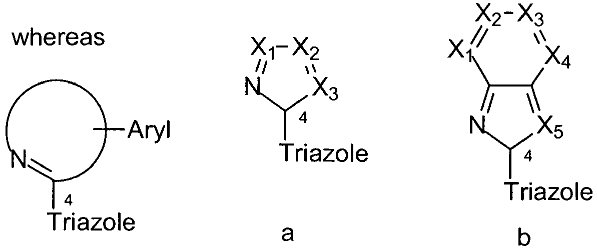

Beispiele für den Substituenten "a" am Triazol sind:
Oxazol Derivate, wobei X₃ = O oder X₂ = O, und alle anderen Reste vom Typ -C-R sind;
Thiazol Derivate, wobei X₃ = S oder X₂ = S, und alle anderen Reste vom Typ -C-R sind.
Isoaxzol Derivate, wobei X₁ = O und alle anderen Reste vom Typ -C-R sind.
Isothiazol Derivate, wobei X₁ = S, und alle anderen Reste vom Typ -C-R sind.
Imidazol Derivate, wobei X₁, X₂ Reste vom Typ -C-R sind und X₃ ein Rest vom Typ N-R ist.
Pyrazol Derivate, wobei X₂, X₃ Reste vom Typ C-R und X₁ ein Rest des Typs N-R ist.
Tetrazol Derivate, wobei X₁, X₂, X₃ alle = N sind.

Beispiele für den Substituenten "b" am Triazol sind:
Benzimidazol Derivate, wobei X₅ vom Typ N-R und X₁, X₂, X₃, X₄ Reste vom Typ -C-R sind. Weitere Stickstoffatome können in den angegliederten Benzolring enthalten sein, somit entstehen Benzimidazolanaloge Pyridin-, Pyrimidin-, Pyrazin- oder Pyridazin -Ring, durch Substitution des C-R durch Stickstoff. Beispielsweise sind Purin-Derivate: X₅ ist ein Rest des Typs N-R und X₁, X₃, sind vom Typ N und X₄ sind vom Typ -C-R.
Alle Substituenten R können unabhängig voneinander H, Methyl-, Ethyl- oder generell lineare oder verzweigt, kondensierte (Decahydronaphthyl-, Adamantyl-), cyclische (Cyclohexyl-) oder ganz oder teilweise substituierte Alkylreste (C1-C20) sein. Die Alkylgruppen können funktionelle Gruppen wie Ether (Ethoxy-, Methoxy-, etc.), Ester-, Amid-, Carbonate etc. or Halogene, bevorzugt F sein. R ist nicht auf Reste vom Alkyl-Typ beschränkt, sondern kann substituierte oder unsubstituierte aromatische Systeme wie Phenyl, Biphenyl, Naphthyl, Phenanthryl etc. und Benzyl etc haben.

Eine Zusammenstellung von grundlegenden aromatischen Systemen wird in der nachfolgenden Tabelle gezeigt.

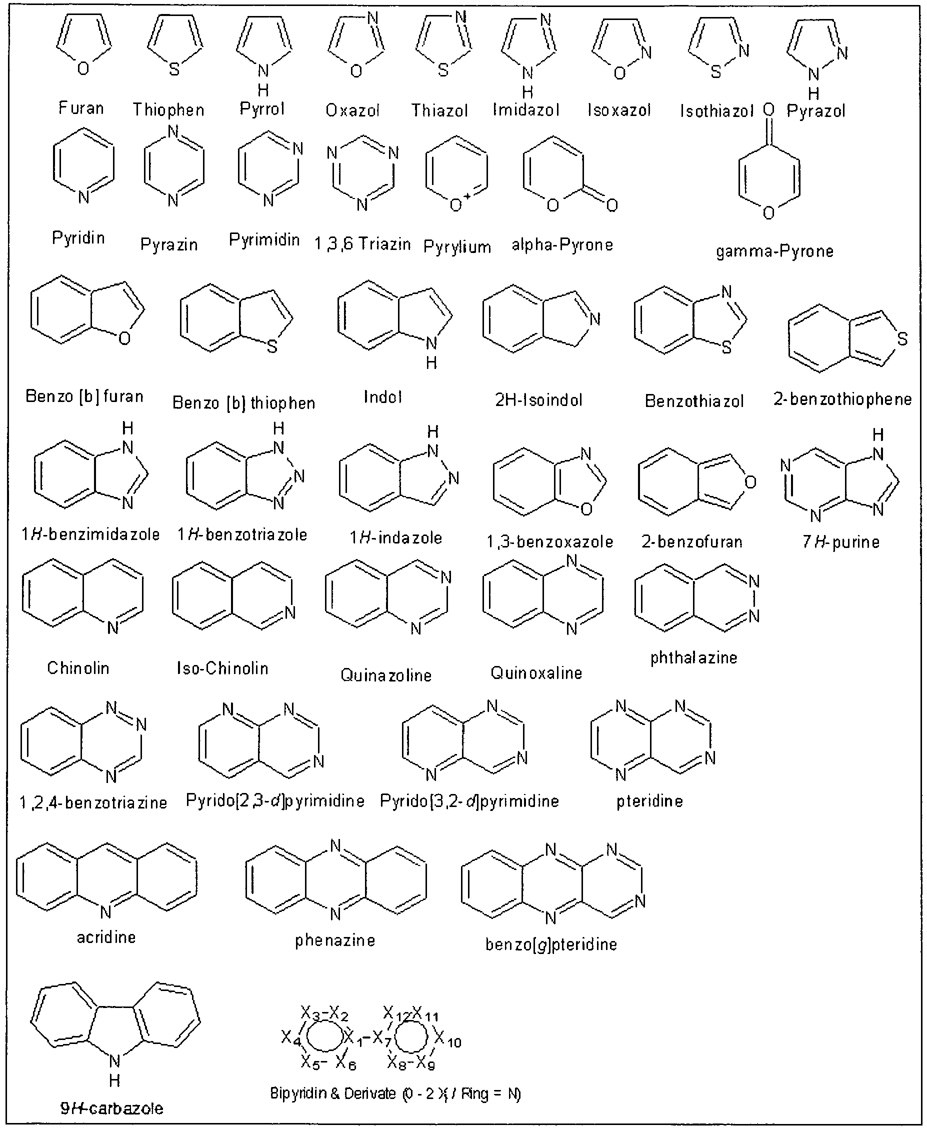

Hier wurden der Einfachheit halber nur die grundlegenden Strukturen gezeigt. Substitutionen können hier an jeder Position mit einer potentiellen Bindungsvalenz auftreten.

Ebenso gut kann der Rest R organometallischer Natur sein, beispielsweise Ferrocenyl-, Phtalacyaninyl- oder Metallkationen, beispielsweise von einem funktionalisierten Kronenether, wie unten gezeigt, umgeben.

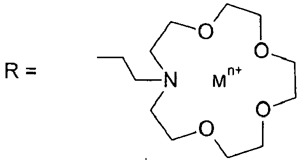

Schließlich kann der Rest R auch geladen sein und somit entweder Ladung in einen bis dato ungeladenen Komplex bringen, was für OLEEC-Anwendungen vorteilhaft ist, oder einen geladenen Komplex neutralisieren und ihn damit für OLED Anwendungen zugänglich machen.

Beispiele für geladene Reste R sind:

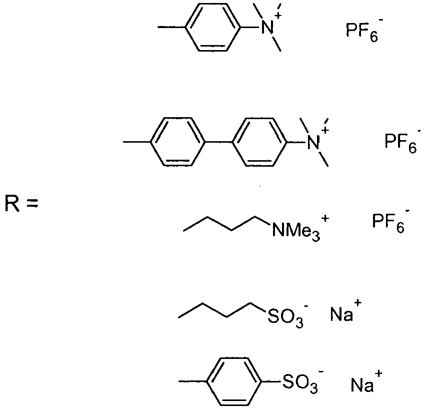

Zur Synthese der 1,2,3-Triazole gibt es verschiedene Ansätze, wobei einige hier über Zitate zum Gegenstand der vorliegenden Beschreibung gemacht werden:

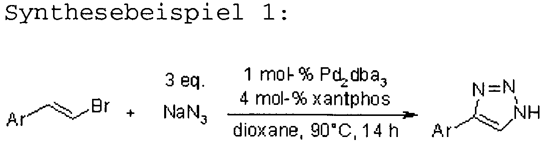

Die Palladium-katalysierte Synthese des 1H-Triazoles aus Alkenyl-Halogenen und NatriumAzid ist eine komplett neue Reaktion im Zusammenhang mit Palladium-Chemie. Siehe dazu J. Barluenga, C. Valdes, G. Beltrán, M. Escribano, F. Aznar, Angew. Chem. Int. Ed., 2006, 45, 6893-6896.

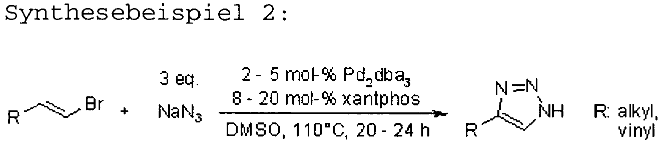

J. Barluenga, C. Valdes, G. Beltrán, M. Escribano, F. Aznar, Angew. Chem. Int. Ed., 2006, 45, 6893-6896.

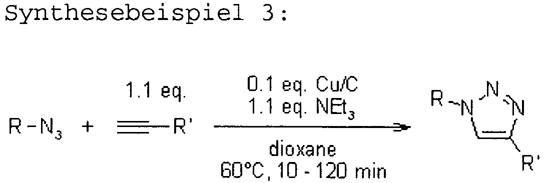

Dies ist eine hocheffektive Chemie zwischen Aziden und endständigen Alkinen, die heterogen durch Kupfer-nanopartikel auf spezielle Kohle katalysiert werden kann. Die Reaktion kann durch den stöchiometrischen Zusatz von Et₃N, durch Temperaturerhöhung oder durch Einsatz von Mikrowelle beschleunigt werden.
B. H. Lipshutz, B. R. Taft, Angew. Chem. Int. Ed. , 2006, 45, 8235-8238.

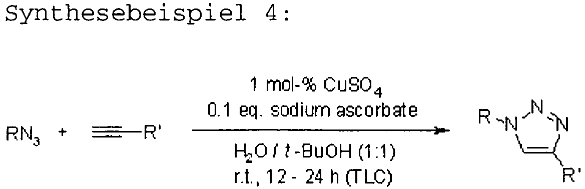

Die Kupfer-katalysierte stufenweise Cacloaddition von Aziden an terminale Alkine eröffnet ein breites Spektrum und ermöglicht die Herstellung von 1,4 disubstituierten 1,2,3-Triazolen in hohen Ausbeuten und mit hoher Regioselektivität.

V. V. Rostovtsev, L. G. Green, V. V. Fokin, K. B. Sharpless, Angew. Chem., 2002, 114, 2708-2711. 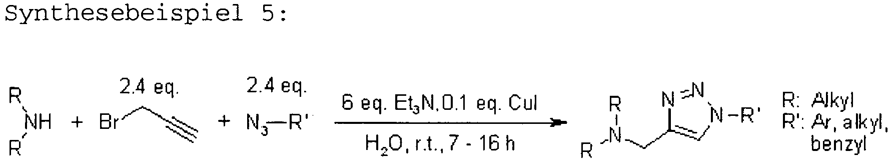 Eine Kupfer (I) katalysierte drei-Komponentenreaktion von Aminen mit Propargyl Halogeniden und Aziden führt in Wasser zu 1-substituierten-1H-1,2,3-triazol-4-ylmethyl)-dialkyl-aminen. Ein sysnthetischer Vorteil ist neben der hohen Selektivität die geringe Umweltbelastung ein breites Feld an Edukten (substrate scope) sowie milde Reaktionsbedingungen und gute Ausbeuten.
Z.-Y. Yan, Y.-B. Zhao, M.-J. Fan, W.-M. Liu, Y.-M. Liang, Tetrahedron, 2005, 61, 9331-9337.

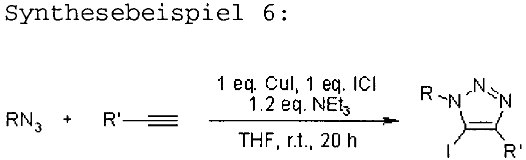

Dies ist eine Methode für die regiospezifische Synthese von 1,4,5-trisubstituierten-1,2,3-Triazol, die durch Kupfer (I) - Jodid katalysiert wird. Das ist das erste Beispiel für eine regiospezifische Synthese von 5-iodo-1,4-disubstituierten-1,2,3-Triazolen, die noch weiterentwickelt werden kann, so dass 1,4,5-trisubstituierte-1,2,3-Triazol-Derivate resultieren.
Y.-M. Wu, J. Deng, Y. L. Li, Q.-Y. Chen, Synthesis, 2005, 1314-1318.

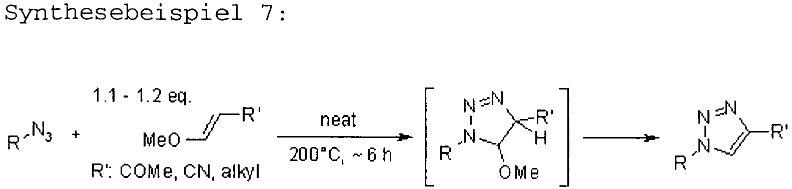

1,2,3-Triazole wurden in mittleren bis guten Ausbeuten durch die Cycloaddition von Alkyl Aziden auf Enolethern unter lösungsmittelfreien Bedingungen hergestellt. Diese Reaktion kann den Zugang zu ringannelierten Triazolen, die über Alkin-Azid-Cycloadditionen unzugänglich sind, eröffnen. Zudem kann die Reaktion leicht vom Labormaßstab hochskaliert werden.Die so hergestellten 1,2,3-Triazole können leicht derivatisiert werden.

D. R. Rogue, J. L. Neill, J. W. Antoon, E. P. Stevens, Synthesis, 2005, 2497-2502.

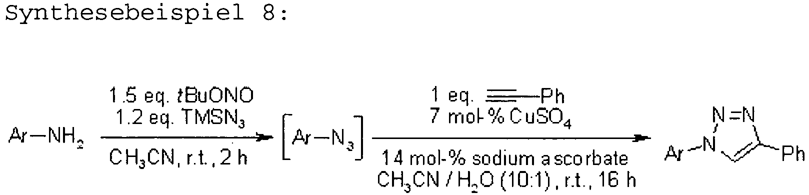

Die Synthese von aromatischen Aziden aus den korrespondierenden Aminen wird unter milden Bedingungen durchgeführt mit tert.-Butyl-Nitrit und Azidotrimethylsilan. 1,4-disubstituierte 1,2,3-Triazole von verschiedensten aromatischen Aminen können so in excellenten Ausbeuten erhalten werden, ohne dass die Azid-Zwischenstufe isoliert werden muss.

K. Barral, A. D. Moorhouse, J. E. Moses, Org. Lett., 2007, 9, 1809-1811.

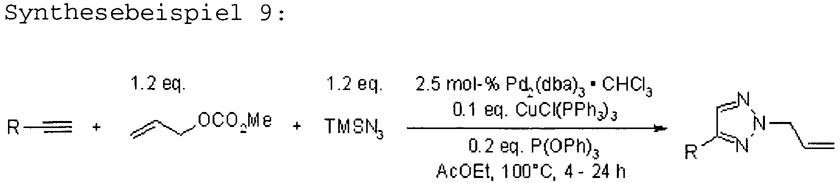

Es wurden Triazole über eine dreikomponentige Kupplungsreaktion mit einem inaktivierten endständigen Alkin, einem Allylcarbonat und einem Trimethylsilyl-Azid unter Palladium (0)-und Kupfer(I) bimetallischen Katalyse hergestellt. Die Deallylation der erhaltenen Triazole wird auch beschrieben.
S. Kamijo, T. Jin, Z. Huo, Y. Yamamoto, J. Am. Chem. Soc., 2003, 125, 7786-7787.

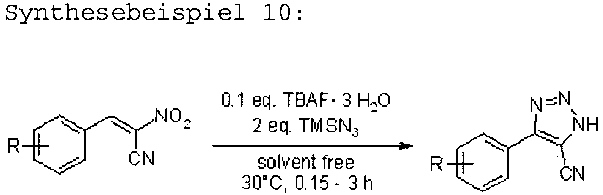

Es handelt sich um eine TBAF-katalysierte [3+2] Cycloaddition eines 2-aryl-1-cyano- oder 2-aryl-1-carbethoxy-1-nitroethen mit TMSN₃ unter lösungsmittelfreien Bedingungen, die die Herstellung von 4-aryl-5-cyano- oder 4-aryl-5-carbethoxy-1*H-*1,2,3-Triazolen unter milden Reaktionsbedingungen mit guten bis ausgezeichneten Ausbeuten ermöglicht.

D. Amantini, F. Fringuelli, O. Piermatti, F. Pizzo, E. Zunino, L. Vaccaro, J. Org. Chem., 2005, 70, 6526-6529. Oder:

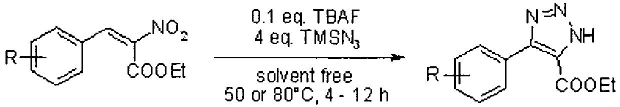

D. Amantini, F. Fringuelli, O. Piermatti, F. Pizzo, E. Zunino, L. Vaccaro, J. Org. Chem., 2005, 70, 6526-6529.

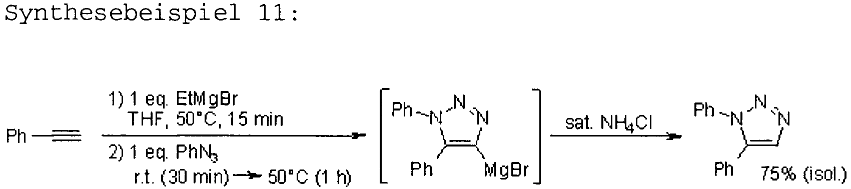

Über sehr effektive Cycloadditionen wurden Triazol-basierte Monophosphin- Liganden hergestellt.
Palladium Komplexe daraus sind sehr effektive Katalysatoren für die Suzuki-Miyaura-Kupplungsreaktion sowie die Aminierungsreaktionen von Arylchloriden.
D. Liu, W. Gao, Q. Dai, X. Zhang, Org. Lett., 2005, 7, 4907-4910.

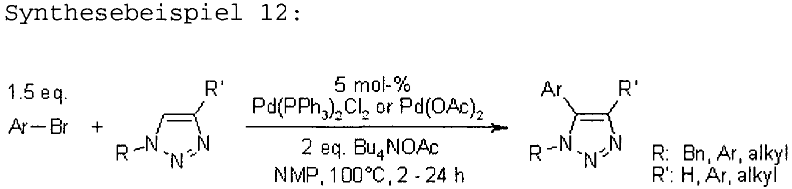

Eine hocheffiziente Methode zur Synthese von multisubstituierten 1,2,3-Triazolen über eine direkte Palladiumkatalysierte C-5 Arylierungsreaktion wird vorgestellt.

S. Chuprakov, N. Chernyak, A. S. Dudnik, V. Gevorgyan, Org. Lett., 2007, 9, 2333-2336.

Im Weiteren werden noch beispielhaft einzelne Synthesebeispiele im Detail beschrieben.

### Beispiel 1: Synthese von

### [F2(ppy)Ir(adamantyltryazolylpyridin)]BF4:

### a) Herstellung des Liganden:

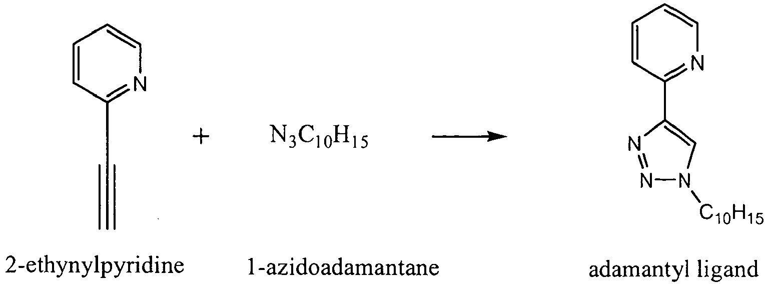

Beschreibung: 1 Äquivalent einer Azid-Komponente und 1 Äquivalent 2-Ethylpyridin werden mit einer katalytischen Menge an Kupferbromid und Pentamethyldiethylentriamin (beide mit ca. 0,04 Äquivalenten) in einem frisch destillierten sauerstofffreien Tetrahydrofuran (6 ml) gerührt. Die Mischung reagiert 12 Stunden bei Raumtemperatur unter Stickstoffatmosphäre ab. Nach dem Entfernen des Lösungsmittels unter reduziertem Druck wird der Feststoff über Säulenchromatographie in Hexan/Ether 20/80 als mobiler Phase, gereinigt. Man erhält eine weiße kristalline Verbindung.

In Figur 1 wird das ¹H-Protonenspektrum der Verbindung gezeigt.

¹H NMR (300 MHz, CD2Cl2) δ 8.54 (d, *J* = 4.2, 1H), 8.23 (s, 1H), 8.13 (d, *J =* 8.0, 1H), 7.77 (t, *J =* 9.0, 1H), 7.24-7.18 (m, 1H), 2.29 (s, 9H), 1.82 (s, 6H). HRMS berechnet für (C17H20N4)H 281.1761 [MH], gefunden 281.1648.

### b) Umsetzung des Liganden mit der chloroverbrückten Iridium-Ausgangsverbindung.

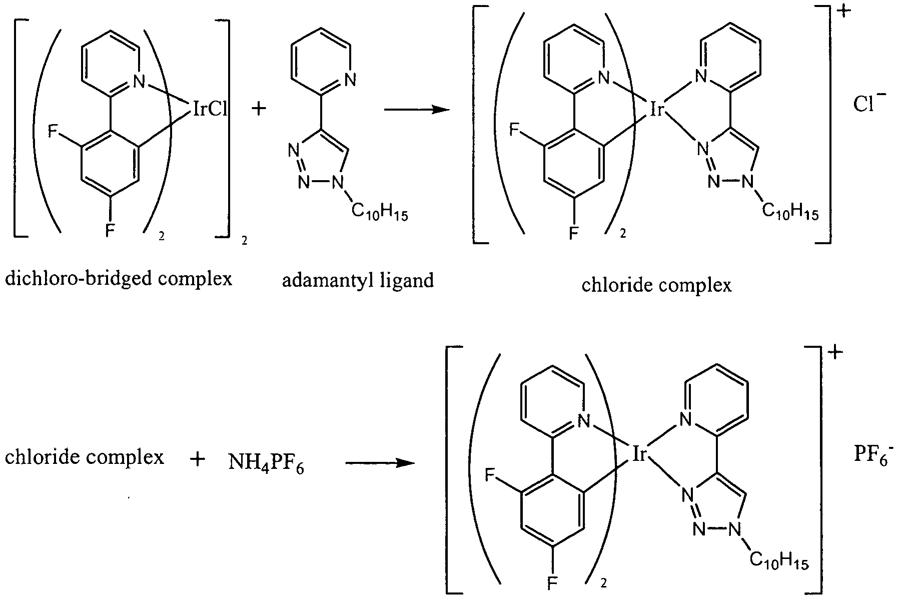

1 Äquivalent des dichloroverbrückten Iridiumkomplexes und 2.2 Äquivalente des Adamantylliganden werden in 30ml Dichloromethan und 10ml Methanol gelöst. Die Mischung wird dann in einen 2-Hals-Rundkolben gegeben und reagiert dort in 4 Stunden bei 45°C unter Stickstoffatmosphäre ab. Nach dem Abkühlen der Mischung auf Raumtemperatur werden die Lösungsmittel unter vermindertem Druck abgezogen und der überschüssige Ligand wird chromatographisch über ein Silicat-Pulver mit EthylAzetat und Methanol als mobiler Phase abgetrennt. Das gereinigte Produkt in Form seines Chlorids wird in Methanol wieder gelöst. Dann wird eine gesättigte Lösung an NH₄PF₆ in Methanol zugegeben. Die Mischung wird für einige Stunden gerührt dann unter vermindertem Druck konzentriert um den gelben Feststoff auszufällen, der dann 3x mit Wasser (3x 20 ml) und 2x mit kaltem Methanol ( 2 x 20 ml) gewaschen wird.

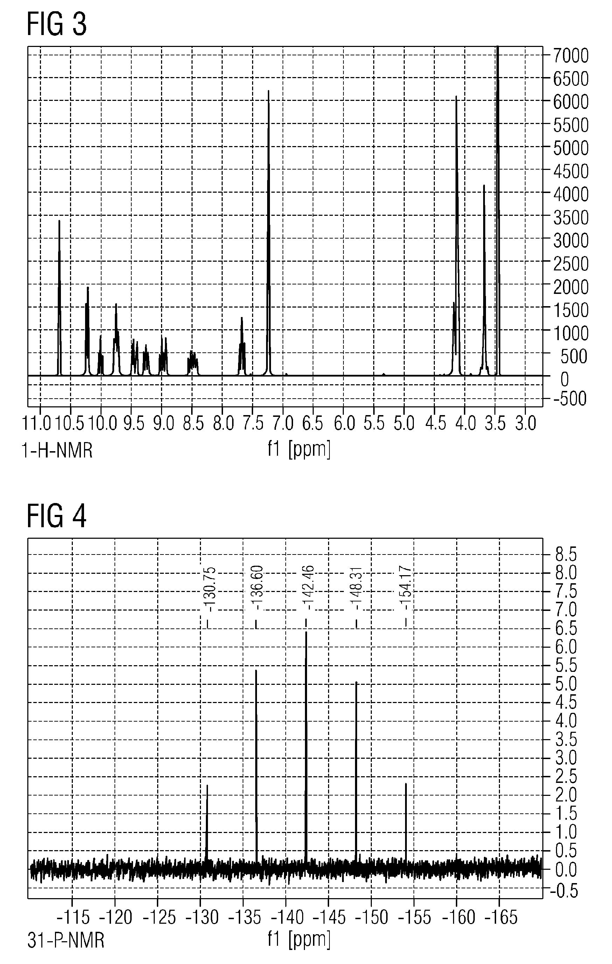

Die Figuren 2 bis 5 zeigen die jeweiligen NMR-Spektren der Verbindung.
¹H NMR (300 MHz, CDCl3) δ 10.69 (s, 1H), 10.27-10.16 (m, 3H), 10.00 (t, *J =* 6, 1H), 9.81-9.70 (m, 3H), 9.46 (d, *J =* 6, 1H), 9.41 (d, J = 6, 1H), 9.28 (t, *J =* 6, 1H), 9.01 (t, *J =* 9.0, 1H), 8.95 (t, *J =* 9.0, 1H), 8.58-8.40 (m, 2H), 7.66 (t, J = 9, 2H), 4.13 (s, 9H), 3.69 (s, 6H). HRMS berechnet für C₃₉H₃₂F₄IrN₆ 853.2254 [M-PF₆], gefunden 853.2171.

### c) Umsetzung des Chlorids zum Tetrafluoroborat:

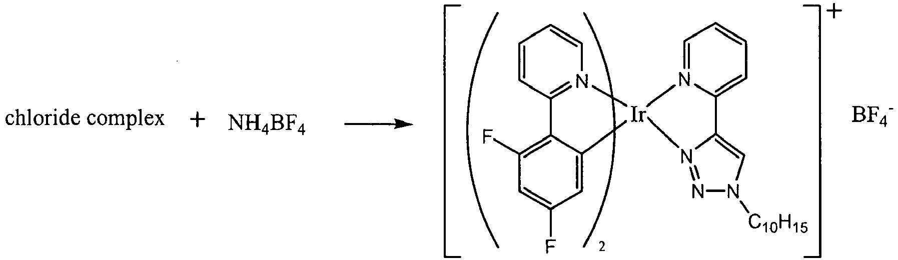

Um das Tetrafluoroborat zu erhalten, wird ein Äquivalent des Chlorid-Komplexes, der wie oben beschrieben erhalten werden kann, in Azeton gelöst. Zu dieser Lösung werden 3 Äquivalente Ammoniumtetrafluoroborat, die in einem Minimum an Wasser gelöst vorliegen, zugegeben. Die Mischung wird über Nacht gerührt, dann wird ein weißes Pulver abfiltriert, das vermutlich ein Überschuss an Ammoniumtetrafluoroborat ist, und das Lösungsmittel unter vermindertem Druck abgezogen. Der erhaltene Feststoff wird teilweise in Wasser gelöst und der unlösliche Teil wird filtriert und mehrmals mit Wasser gewaschen. Schließlich wird er in Dichloromethan gelöst und über Magnesiumsulfat getrocknet.

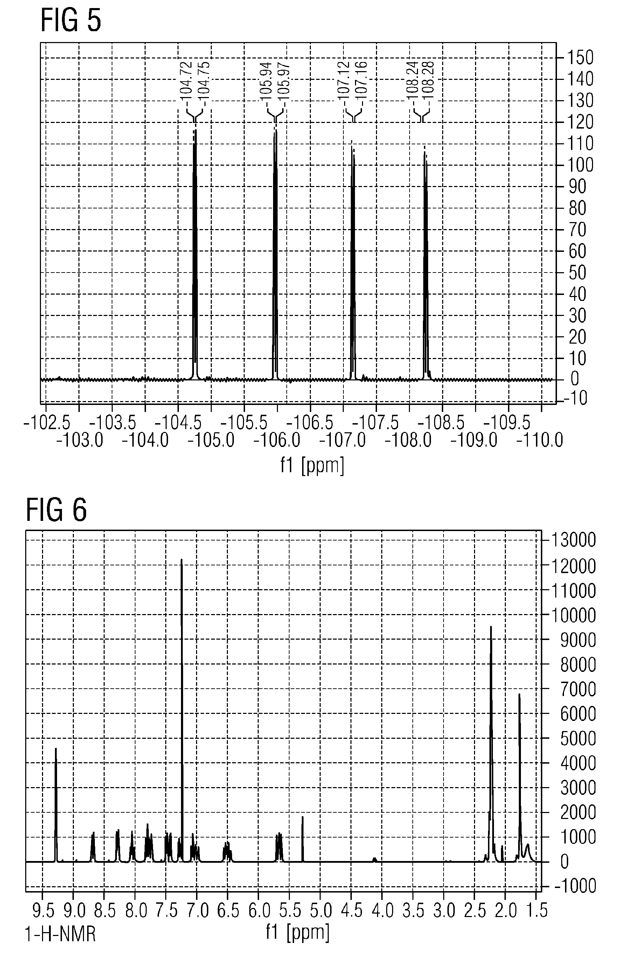

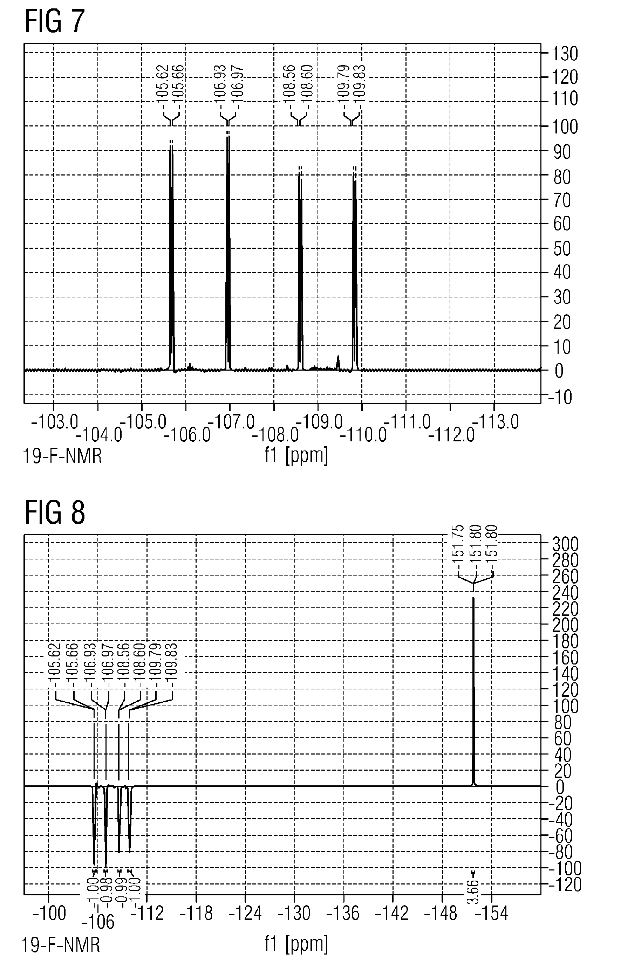

Figur 6 bis 8 zeigen die NMR-Spektren des Tetrafluoroborats.

¹H NMR (300 MHz, CDCl3) δ 9.28 (s, 1H), 8.68 (d, J = 8.0, 1H), 8.28 (d, *J =* 8.0, 2H), 8.05 (t, J = 7.1, 1H), 7.80 (t, J = 9.0, 2H), 7.73 (d, *J =* 3.0, 1H), 7.50 (d, *J =* 6.0, 1H), 7.44 (d, *J =* 6.0, 1H), 7.29 (t, *J =* 6.0, 1H), 7.08 (t, *J =* 6.0, 1H), 7.01 (t, *J =* 6.0, 1H),6.59-6.44 (m, 2H), 5.72 - 5.60 (m, 2H), 2.19 (s, 9H), 1.72 (s, 6H). HRMS berechnet für C₃₉H₃₂F₄IrN₆ 853.2254 [M-BF₄], gefunden 853.2148.

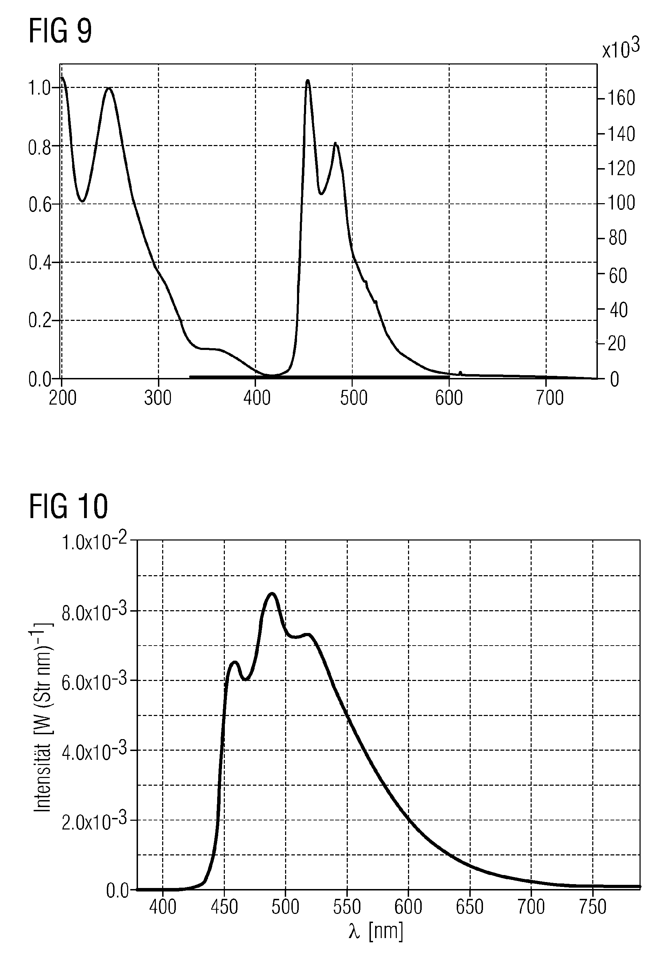

Figuren 9 bis 10 zeigen Spektren von [F2(ppy)Ir(adamantyltry-azolylpyridin)]PF6; ein Photolumineszenz-Spektrum (Figur 9) und ein Elektrolumineszenzspektrum (Figur 10).

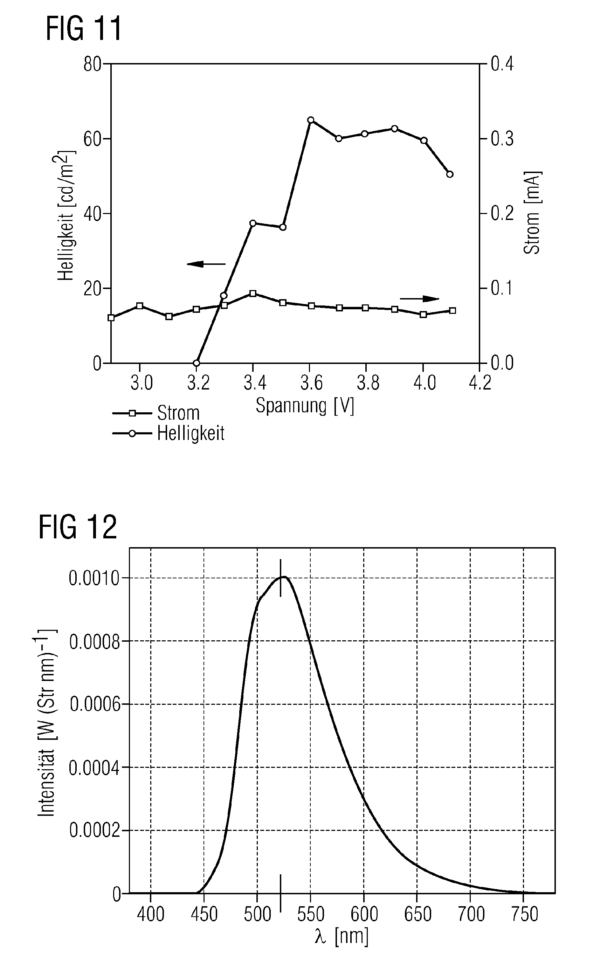

Figur 11 zeigt die Licht-Strom-Spannungs-Charakteristik der Verbindung [F2(ppy)Ir(adamantyltryazolylpyridin)]PF6.

Figur 12 zeigt ein Elektrolumineszenz Spektrum der Tetrafluoroborat-Verbindung [F2(ppy)Ir(adamantyltryazolylpyridin)]BF4.

Figur 13 zeigt wieder eine Licht- Strom- Spannungs- Charakteristik, diesmal der Verbindung [F2(ppy)Ir(adamantyltryazolyl-pyridin)]BF4.

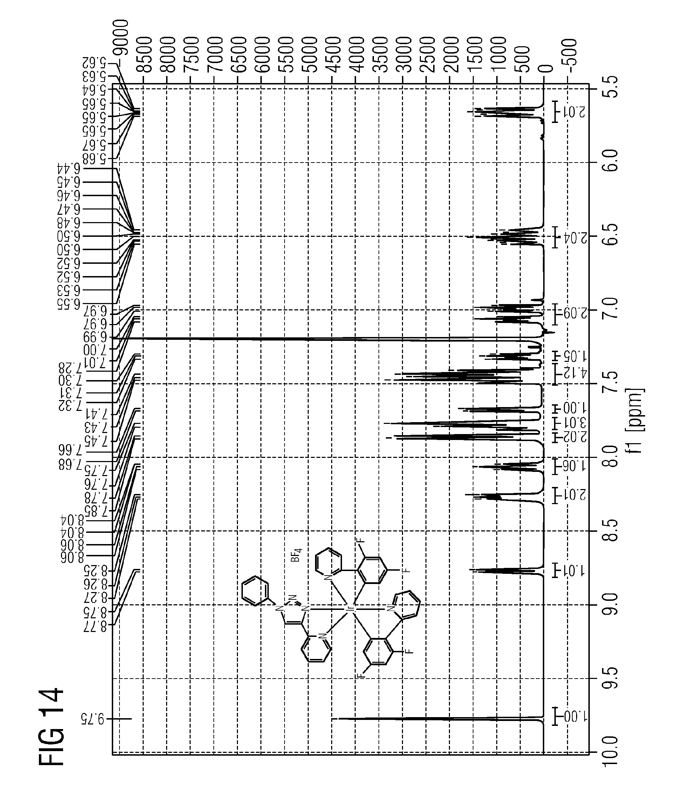

Figur 14 zeigt das 1-H- NMR für die Verbindung Bis-(2,4-di-fluorophenyl-pyridyl) (4-pyridyl-1-phenyl-triazole) iridium (III)-tetrafluorobrat.

**In Tabelle 1 werden die Redoxpotentiale der verbrückten Iridium (III) Verbindungen gezeigt.**

| Compound | E_{1/2}^{Red} (V) | E_{1/2}^{Ox} (V) |
|---|---|---|
| **Flu** | -1.72; -2.06 | - |
| **Ir-flu-Ir** | -2.05^{b} | 0.88 |
| **FFIr-flu-IrFF** | -1.96^{b} | 1.21 |
| **Ru-flu-Ru** | -1.76; -1.94^{c} | 0.93 |
| ^{a}.Scanrate 100mV/s | | |
| ^{b} Irreversibel. ^{c} Adsorption an der Elektrode. | | |

Die Messungen wurden in wasserfreiem Acetonitril durchgeführt (für die Komplexe), und in THF für die Liganden, (Flu) die Werte wurden gegenüber Ferrocen/Ferrocenium als internem Standard gemessen.

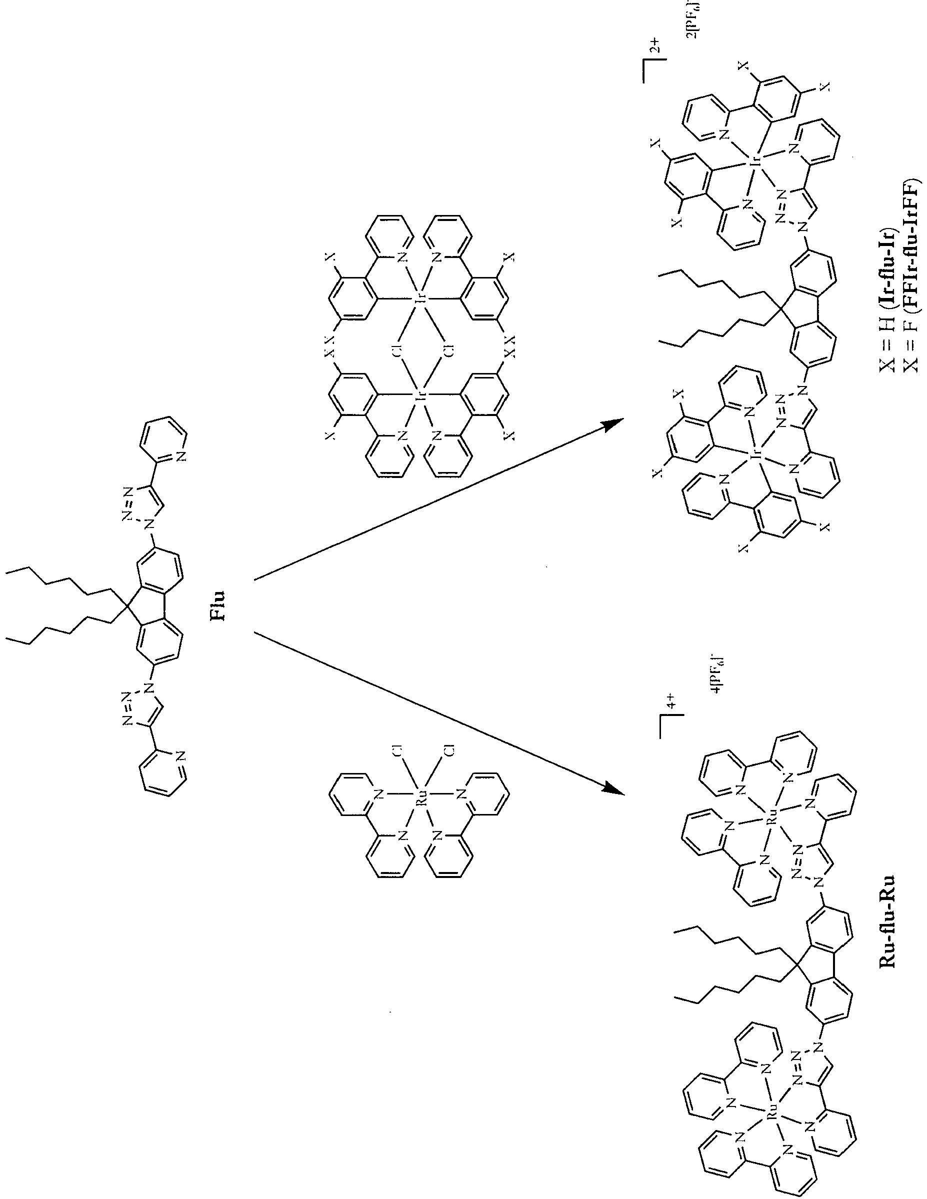

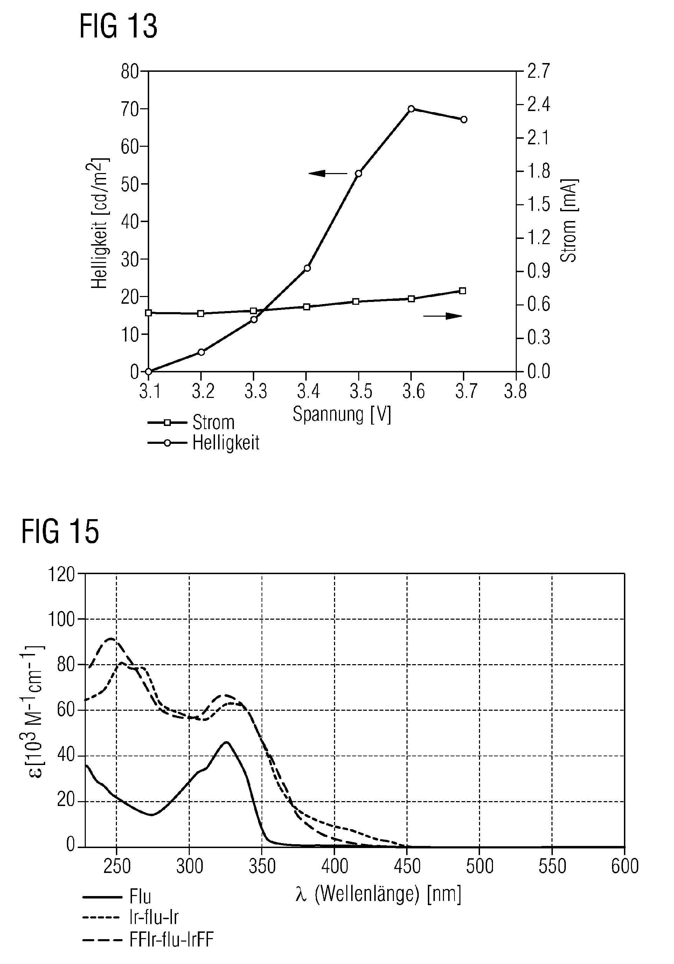

Figur 15 zeigt ein Absorptionsspektrum einer verbrückten Iridium (III)-Triazol Verbindung.

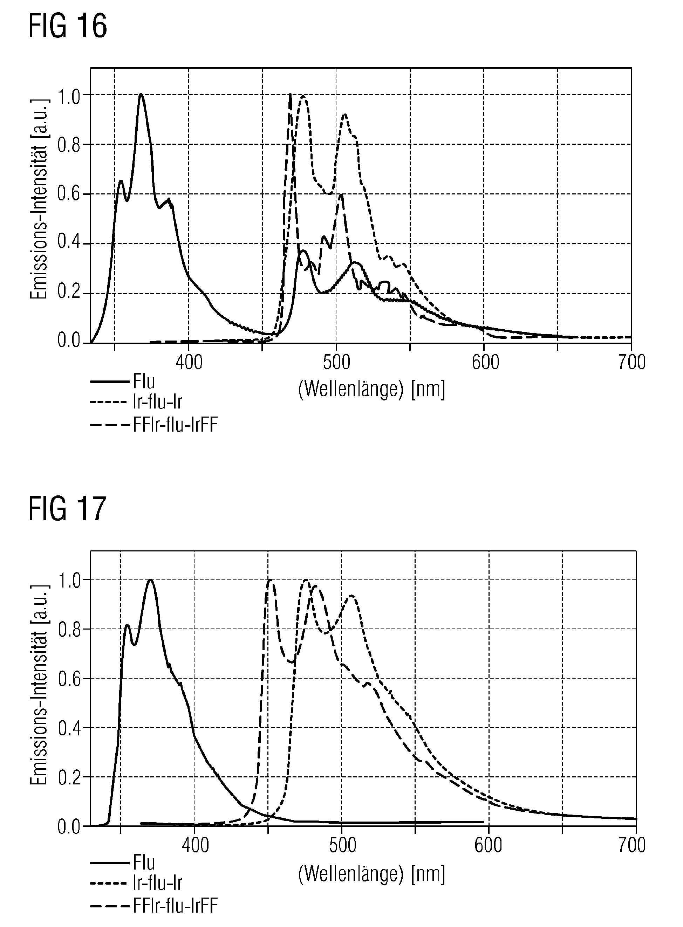

Figur 16 zeigt ein Photolumineszenzspektrum der verbrückten Iridium (III)-Verbindung wie oben beschrieben, bei einer Temperatur von 77 Kelvin und

Figur 17 zeigt ein weiteres Photolumineszenzspektrum der verbrückten Iridium (III)-Verbindung bei Raumtemperatur.

Im Folgenden werden noch Strukturen von zwei Triazol-Liganden gemäß der Erfindung gezeigt, die beispielsweise gemäß der Erfindung eingesetzt werden. Es werden damit kräftig blaue Emitter erzeugt.

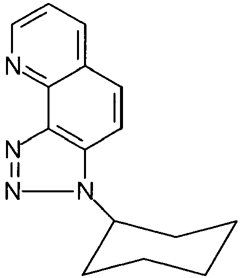

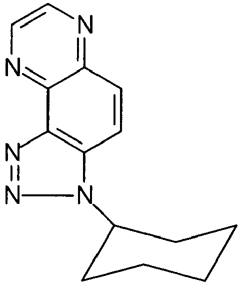

Die vorliegende Erfindung beschreibt ein Triazolligandensystem, das einsetzbar ist zur Erzeugung von blauen und grünen Emittern, die in organischen Licht emittierenden elektrochemischen Zellen OLEECs einsetzbar sind. Einige der hier erstmals gezeigten blauen Emitter, insbesondere die hier vorgestellte Klasse der Iridium-komplexverbindungen, sind die blauesten Emitter, die zurzeit überhaupt existieren.

Die Erfindung betrifft eine lichtemittierende organische elektrochemische Zelle, Verfahren zur Herstellung einer lichtemittierenden organischen elektrochemischen Zelle (organic light emitting electrochemical cell OLEEC).

## Patentansprüche

1. Lichtemittierende organische elektrochemische Zelle umfassend
- ein Substrat,
- eine erste Elektrodenschicht auf dem Substrat,
- zumindest eine organische emittierende Schicht auf der ersten Elektrodenschicht und
eine zweite Elektrodenschicht auf der organischen emittierenden Schicht, wobei die organische emittierende Schicht eine phosphoreszente Metallkomplexverbindung umfasst, die die Strukturformel 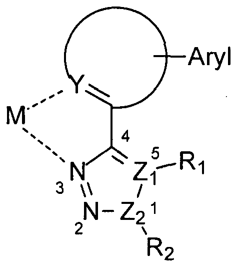 aufweist, wobei gilt:
M=Ir, Re, Os, Pt, Au, Hg, Ru, Rh, Pd, Ag, Cu
Y, Z₁, Z₂ = N oder C
R₁, R₂ = unabhängig voneinander - H, verzweigte Alkylreste, unverzweigte Alkylreste, kondensierte Alkylreste, ringförmige Alkylreste, vollständig oder teilweise substituierte unverzweigte Alkylreste, vollständig oder teilweise substituierte verzweigte Alkylreste, vollständig oder teilweise substituierte kondensierte Alkylreste, vollständig oder teilweise substituierte ringförmige Alkylreste, Alkoxygruppen, Amine, Amide, Ester, Carbonate, Aromaten, vollständig oder teilweise substituierte Aromaten, Heteroaromaten, kondensierte Aromaten, vollständig oder teilweise substituierte kondensierte Aromaten, Heterocyclen, vollständig oder teilweise substituierte Heterocyclen, kondensierte Heterocyclen, Halogene, Pseudohalogene, wobei R₁ und/oder R₂ geladen sind, und damit die phosphoreszente Metallkomplexverbindung geladen ist,
und
Aryl = ein beliebiger, teilweise oder vollständig substituierter aromatischer oder heteroaromatischer Rest ist, der auch kondensiert sein kann, eine Brücke zu einer weiteren Verbindung knüpfen kann, und/oder kondensiert oder anneliert mit weiteren Aromaten oder Heteroaromaten, sowie verbunden mit weiteren cyclischen Verbindungen, vorliegen kann.

2. Lichtemittierende organische elektrochemische Zelle nach Anspruch 1, wobei die phosphoreszente Metallkomplexverbindung eine Triazol-Einheit aufweist, welche ausgewählt ist aus der Gruppe der 1,2,3-Triazole und der 1,2,4 Triazole.

3. Lichtemittierende organische elektrochemische Zelle nach einem der Ansprüche 1 oder 2, wobei die phosphoreszente Metallkomplexverbindung verbrückt ist.

4. Lichtemittierende organische elektrochemische Zelle nach Anspruch 2, wobei bei der phosphoreszenten Metallkomplexverbindung die Triazol-Einheit in 4-Stellung substituiert ist.

5. Lichtemittierende organische elektrochemische Zelle nach Anspruch 2, wobei bei der phosphoreszenten Metallkomplexverbindung die Triazoleinheit in 4-Stellung Aryl-substituiert ist.

6. Lichtemittierende organische elektrochemische Zelle nach Anspruch 1, wobei die phosphoreszente Metallkomplexverbindung eine Verbindung mit der Strukturformel Ia 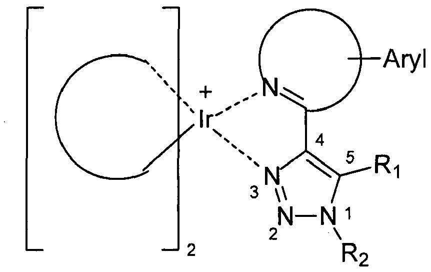 umfasst.

7. Lichtemittierende organische elektrochemische Zelle nach Anspruch 1, wobei R₁ und/oder R₂ zusätzlich an M koordiniert sind.

8. Lichtemittierende organische elektrochemische Zelle nach Anspruch 1, wobei die phosphoreszente Metallkomplexverbindung folgende Strukturformeln aufweist: 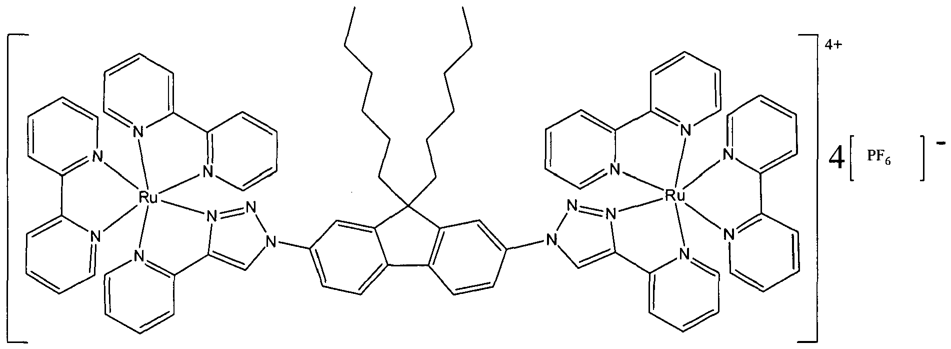 oder 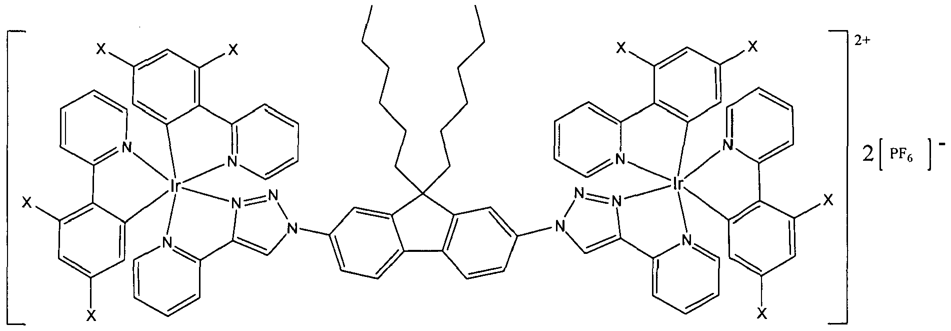 , wobei X H oder F ist.

9. Lichtemittierende organische elektrochemische Zelle nach Anspruch 1, wobei die phosphoreszente Metallverbindung in einem Matrixmaterial vorhanden ist.

10. Lichtemittierende organische elektrochemische Zelle nach Anspruch 1, die bei Anlegen einer Spannung Licht einer Farbe emittiert, die ausgewählt ist aus einer Gruppe umfassend die Farben grün, blau-grün, hellblau, tiefblau, blau.

11. Lichtemittierende organische elektrochemische Zelle nach Anspruch 1, wobei das Substrat und die erste Elektrodenschicht transparent sind.

12. Lichtemittierende organische elektrochemische Zelle nach einem der vorhergehenden Ansprüche, wobei R₁ und/oder R₂ aus folgender Gruppe ausgewählt ist:

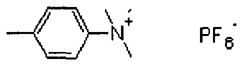

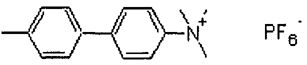

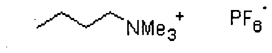

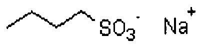

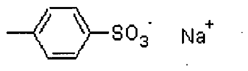

13. Verfahren zur Herstellung einer lichtemittierenden organischen elektrochemischen Zelle gemäß den Ansprüchen 1 bis 12 beinhaltend die folgenden Verfahrensschritte zur Herstellung einer phosphoreszenten Metallkomplexverbindung
A) Bereitstellen einer Zentralatomverbindung eines metallischen Zentralatoms, aufweisend an das Zentralatom koordinierte Austauschliganden,
B) Mischen der Zentralatomverbindung und eines in einem ersten Lösungsmittel gelösten Liganden zur Bildung der Metallkomplexverbindung, wobei der Austauschligand durch den Liganden, der zweizähnig am Zentralatom koordiniert und eine Triazoleinheit umfasst, ersetzt wird.

14. Verfahren nach Anspruch 13, wobei die Metallkomplexverbindung säulenchromatographisch gereinigt wird.

## Claims

1. Light-emitting organic electrochemical cell comprising
- a substrate,
- a first electrode layer on the substrate,
- at least one organic emitting layer on the first electrode layer and
a second electrode layer on the organic emitting layer, wherein the organic emitting layer comprises a phosphorescent metal complex having the structural formula 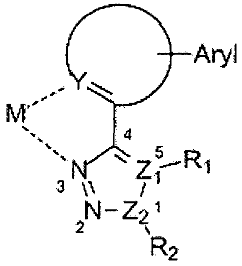 where:
M = Ir, Re, Os, Pt, Au, Hg, Ru, Rh, Pd, Ag, Cu, Y, Z₁, Z₂ = N or C,
R₁, R₂ = independently H, branched alkyl radicals, unbranched alkyl radicals, fused alkyl radicals, cyclic alkyl radicals, fully or partly substituted unbranched alkyl radicals, fully or partly substituted branched alkyl radicals, fully or partly substituted fused alkyl radicals, fully or partly substituted cyclic alkyl radicals, alkoxy groups, amines, amides, esters, carbonates, aromatic systems, fully or partly substituted aromatic systems, heteroaromatic systems, fused aromatic systems, fully or partly substituted aromatic systems, heterocycles, fully or partly substituted heterocycles, fused heterocycles, halogens, pseudohalogens, where R₁ and/or R₂ are charged, and hence the phosphorescent metal complex is charged,
and
aryl = any partly or fully substituted aromatic or heteroaromatic radical which may also be fused, may form a bridge to a further compound, and/or may be fused and/or annelated to further aromatic or heteroaromatic systems, and bonded to further cyclic compounds.

2. Light-emitting organic electrochemical cell according to Claim 1, wherein the phosphorescent metal complex has a triazole unit selected from the group of the 1,2,3-triazoles and the 1,2,4-triazoles.

3. Light-emitting organic electrochemical cell according to either of Claims 1 and 2, wherein the phosphorescent metal complex is bridged.

4. Light-emitting organic electrochemical cell according to Claim 2, wherein the triazole unit in the phosphorescent metal complex is 4-substituted.

5. Light-emitting organic electrochemical cell according to Claim 2, wherein the triazole unit in the phosphorescent metal complex is 4-aryl-substituted.

6. Light-emitting organic electrochemical cell according to Claim 1, wherein the phosphorescent metal complex comprises a compound having the structural formula Ia

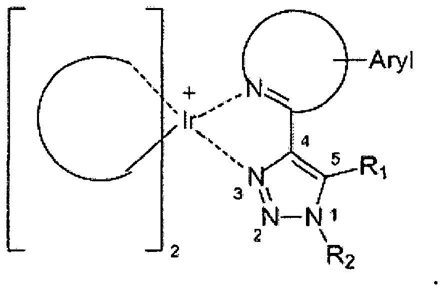

7. Light-emitting organic electrochemical cell according to Claim 1, wherein R₁ and/or R₂ is/are additionally coordinated to M.

8. Light-emitting organic electrochemical cell according to Claim 1, wherein the phosphorescent metal complex has the following structural formulae: 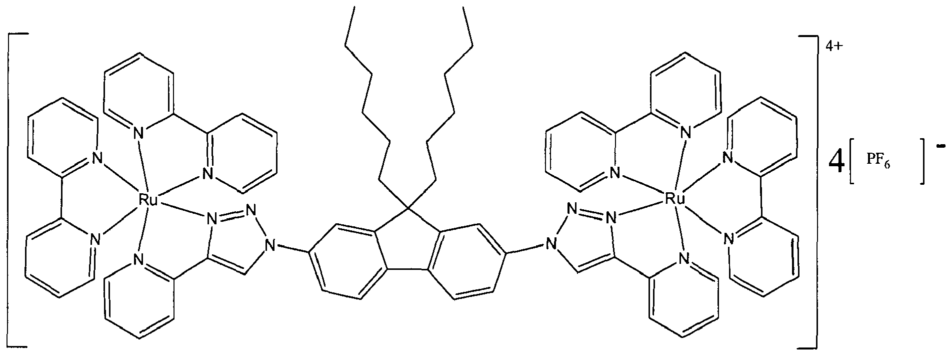 or 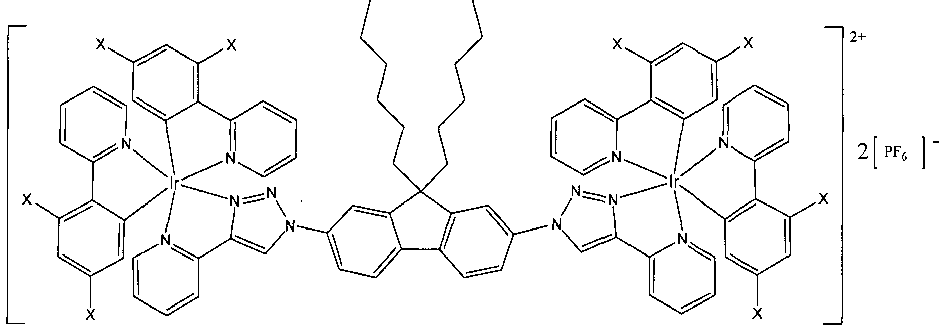 where X is H or F.

9. Light-emitting organic electrochemical cell according to Claim 1, wherein the phosphorescent metal compound is present in a matrix material.

10. Light-emitting organic electrochemical cell according to Claim 1 which, on application of a voltage, emits light of a colour selected from a group comprising the colours green, blue-green, light blue, deep blue, blue.

11. Light-emitting organic electrochemical cell according to Claim 1, wherein the substrate and the first electrode layer are transparent.

12. Light-emitting organic electrochemical cell according to any of the preceding claims, wherein R₁ and/or R₂ is/are selected from the following group:

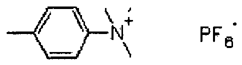

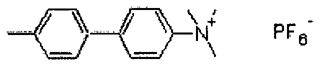

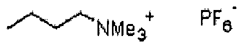

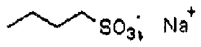

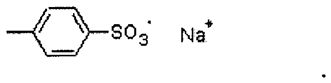

13. Process for preparing a light-emitting organic electrochemical cell according to Claims 1 to 12, including the following process steps for preparing a phosphorescent metal complex:
A) providing a central atom compound of a metallic central atom, having exchangeable ligands coordinated to the central atom,
B) mixing the central atom compound and a ligand dissolved in a first solvent to form the metal complex, the exchangeable ligand being replaced by the ligand which coordinates to the central atom in a bidentate manner and includes a triazole unit.

14. Process according to Claim 13, wherein the metal complex is purified by column chromatography.

## Revendications

1. Cellule électrochimique organique émettrice de lumière, comprenant :
- un substrat,
- une première couche d'électrode sur le substrat,
- au moins une couche organique émettrice sur la première couche d'électrode et
- une seconde couche d'électrode sur la couche organique émettrice, la couche organique émettrice comprenant un composé de complexe métallique phosphorescent, qui présente la formule structurale 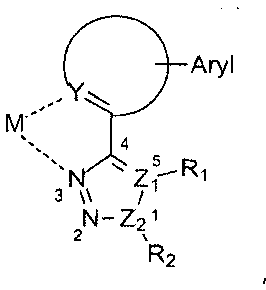 dans laquelle :
M = Ir, Re, Os, Pt, Au, Hg, Ru, Rh, Pd, Ag, Cu Y, Z₁, Z₂ = N ou C
R₁, R₂ = indépendamment l'un de l'autre - H, radicaux alkyle ramifiés, radicaux alkyle non ramifiés, radicaux alkyle condensés, radicaux alkyle cycliques, radicaux alkyle non ramifiés entièrement ou partiellement substitués, radicaux alkyle ramifiés entièrement ou partiellement substitués, radicaux alkyle condensés entièrement ou partiellement substitués, radicaux alkyle cycliques entièrement ou partiellement substitués, groupes alcoxy, amines, amides, esters, carbonates, aromatiques, aromatiques entièrement ou partiellement substitués, hétéroaromatiques, aromatiques condensés, aromatiques condensés entièrement ou partiellement substitués, hétérocycles, hétérocycles entièrement ou partiellement substitués, hétérocycles condensés, halogènes, pseudohalogènes, R₁ et/ou R₂ étant chargés, et le composé de complexe métallique phosphorescent étant par conséquent chargé, et
Aryl = un radical aromatique ou hétéroaromatique quelconque, partiellement ou entièrement substitué, qui peut également être condensé, peut relier un pont vers un autre composé et/ou peut se présenter sous forme condensée ou annelée avec d'autres aromatiques ou hétéroaromatiques, ainsi que reliée avec d'autres composés cycliques.

2. Cellule électrochimique organique émettrice de lumière selon la revendication 1, dans laquelle le composé de complexe métallique phosphorescent comprend une unité triazole, qui est choisie dans le groupe des 1,2,3-triazoles et des 1,2,4-triazoles.

3. Cellule électrochimique organique émettrice de lumière selon la revendication 1 ou 2, dans laquelle le composé de complexe métallique phosphorescent est ponté.

4. Cellule électrochimique organique émettrice de lumière selon la revendication 2, dans laquelle l'unité triazole dans le composé de complexe métallique phosphorescent est substituée en position 4.

5. Cellule électrochimique organique émettrice de lumière selon la revendication 2, dans laquelle l'unité triazole dans le composé de complexe métallique phosphorescent est substituée par aryle en position 4.

6. Cellule électrochimique organique émettrice de lumière selon la revendication 1, dans laquelle le composé de complexe métallique phosphorescent comprend un composé de formule structurale Ia

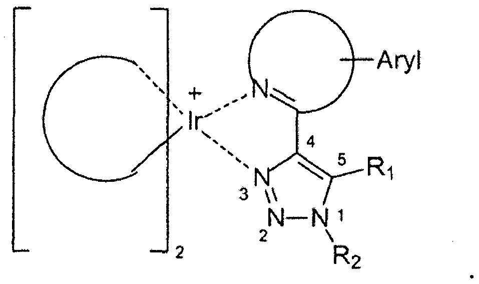

7. Cellule électrochimique organique émettrice de lumière selon la revendication 1, dans laquelle R₁ et/ou R₂ sont également coordonnés à M.

8. Cellule électrochimique organique émettrice de lumière selon la revendication 1, dans laquelle le composé de complexe métallique phosphorescent présente les formules structurales suivantes : 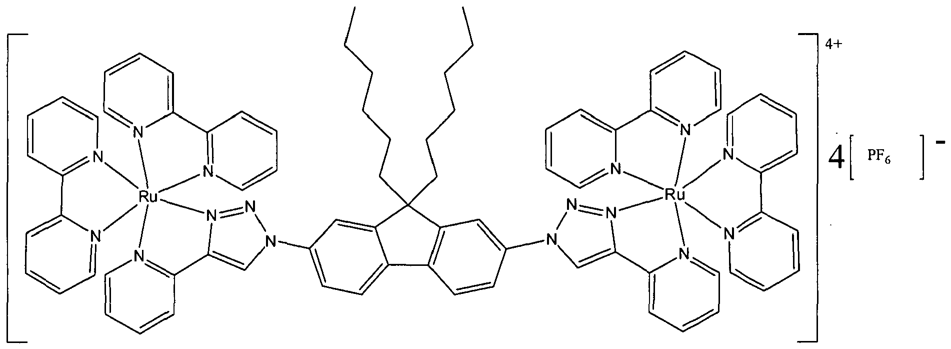 ou 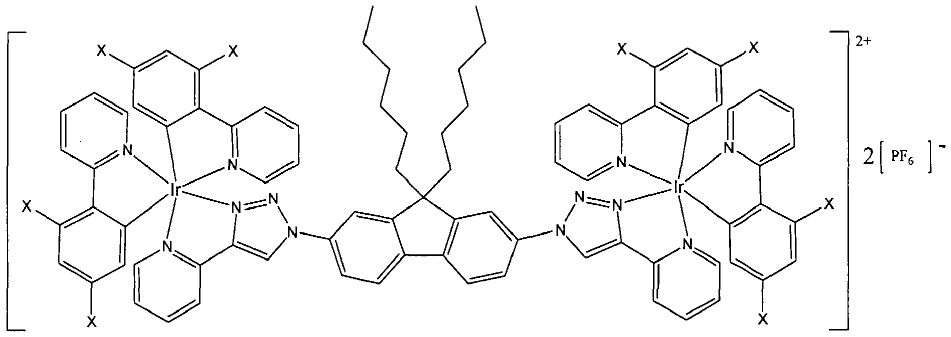 , dans lesquelles X représente H ou F.

9. Cellule électrochimique organique émettrice de lumière selon la revendication 1, dans laquelle le composé métallique phosphorescent est présent dans un matériau de matrice.

10. Cellule électrochimique organique émettrice de lumière selon la revendication 1, qui émet lors de l'application d'une tension une lumière d'une couleur choisie dans un groupe comprenant les couleurs vert, bleu-vert, bleu clair, indigo, bleu.

11. Cellule électrochimique organique émettrice de lumière selon la revendication 1, dans laquelle le substrat et la première couche d'électrode sont transparents.

12. Cellule électrochimique organique émettrice de lumière selon l'une quelconque des revendications précédentes, dans laquelle R₁ et/ou R₂ sont choisis dans le groupe suivant :

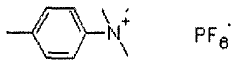

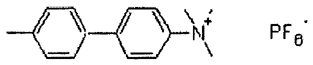

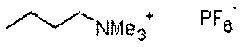

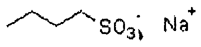

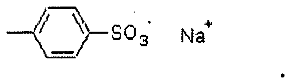

13. Procédé de fabrication d'une cellule électrochimique organique émettrice de lumière selon les revendications 1 à 12, comprenant les étapes de procédé suivantes pour la fabrication d'un composé de complexe métallique phosphorescent :
A) la préparation d'un composé d'atome central d'un atome métallique central, comprenant des ligands d'échange coordonnés à l'atome central,
B) le mélange du composé d'atome central et d'un ligand dissous dans un premier solvant pour la formation du composé de complexe métallique, le ligand d'échange étant remplacé par le ligand qui est coordonné de manière bidentate à l'atome central et comprend une unité triazole.

14. Procédé selon la revendication 13, dans lequel le composé de complexe métallique est purifié par chromatographie sur colonne.
